# EUROPEAN PATENT APPLICATION

(11) **EP 1 958 641 A1**
(43) Date of publication of application: **20.08.2008**
(21) Application number: 07102568.8
(22) Date of filing: 16.02.2007
(51) Int. Cl.: A61K 36/00, A61P 35/00, A61P 29/00

(54) **Treatment and prevention of inflammation**

(71) Applicant: Bionature E.A. Limited, 2406 Egomi, Nicosia (CY)
(72) Inventor: Castanas, Elias, Crete, 71003 Heraklion (GR)
(74) Representative: Rasmussen, Torben Ravn

(57) **Abstract**

Disclosed is the use of a product derived from the plant *Pistachia lentiscus* var. *chia* for the manufacture of a pharmaceutical composition for treating or preventing inflammation and cell proliferation. In a preferred aspect the product is a dilution of the oily steam distillate of the resin. Also disclosed is a pharmaceutical composition comprising the product derived from the plant *Pistachia lentiscus* var. *chia* in a dilution of 10⁻⁵ or less.

## Description

The present invention relates to a new use of a product derived from the plant *Pistachia lentiscus* var. *chia.* Especially, the present invention relate to the use of said product for the manufacture of a pharmaceutical composition for treatment or prevention of inflammation

### Prior art

Inflammation describes the selective or non-selective activation of the immune system in response to a foreign micro-organism or chemical agent. Inflammation can induce the local activation of monocytes or phagocytes, resulting in the production of various pro-inflammatory cytokines. The latter acts in turn as proliferative or activating factors, or chemo-attractants of other inflammatory cells, such as lymphocytes or granulocytes. These cells accumulate at the inflammation site, and decompose the foreign micro-organism or substance. They also further activate the inflammatory response, through the secretion of other (or the same) pro-inflammatory cytokines. In this respect, any pro-inflammatory situation can be assayed by the measurement of produced cytokines.

Table 1 presents cytokines produced by specific cell populations of pro-inflamatory cells. It derives that the system cytokines-pro-inflammatory cells has a discrete autocrine or paracrine effect on the pro-inflamatory cells themselves.

**Table 1: Secretion of cytokines by different pro-inflamatory cell population**

| | Macrophages | Lymphocytes | | | Stroma Cells |
|---|---|---|---|---|---|
| | | Th0 | TH1 | TH2 | |
| IL1 | + | | | | |
| IL2 | | + | + | | |
| IL4 | | | | + | |
| IL5 | | | | + | |
| IL6 | + | | | + | |
| IL7 | | | | | + |
| IL8 | + | | | | |
| IL10 | | | | + | |
| IL12 | + | | | | |
| IL13 | | | | + | |
| INFγ | | | + | | |
| GM-CSF | | | + | | |
| TNFα | + | | | | |

Patent GR1003541 deals with extracts from leaves, product or other parts of the plant *Pistacia lentiscus* var. *Chia.* These extracts have an anti-microbial or anti-fungal activity as a pharmaceutical or para-pharmaceutical product. US patent application No. US2003096020 claims the use of a *Solanum xanthocarpum, Albizzia lebbeck, Tribulus terrestris, Glycyrrhiza glabra, Pistachia integerrima, Adathoda vasica* and *Woodfordia fruticosa.* These products are used as agents for the treatment of bronchial asthma.

Greek patent No GR1003868 and international patent application No W003092712 use neutral or acid extracts of the resin from the plant *Pistacia lentiscus* var. *chia.* These extracts may be used as antioxidants, cytostatic agents, as well as stimulators of wound healing.

In recent years, university researchers have provided the scientific evidence for the medicinal properties of mastic. A 1985 study by the University of Thessaloniki discovered that mastic can reduce bacterial plaque in the mouth by 41.5 percent. A 1998 study by the University of Athens found that mastic oil has antibacterial and anti-fungal properties. Another study performed in 1998 at University of Nottingham, published in the New England Journal of Medicine, found that mastic can heal peptic ulcers.

### Brief description of the invention

The present invention describes the use of a product derived from the plant *Pistachia lentiscus* var. *chia* for the manufacture of a pharmaceutical composition for treating or preventing inflammation.

The plant *Pistacia lentiscus* var. *chia* is an evergreen shrub or small tree growing to 3-4 m tall, mainly cultivated on the Greek island of Chios, but it is also native throughout the Mediterranean region. The product used according to the present invention may relate to any part of the plant. In general, the resin from the plant is best suitable for the preparation of a pharmaceutical composition. The resin may be collected by bleeding it from cuts in the bark of the plant. After the resin has been dried is becomes hard and brittle. The dried resin is also known as mastic.

The active substances of the dried or the non-dried resin may be separated from the remainder by various separation methods, such as extraction and distillation. A preferred separation method is steam distillation as even temperature sensitive compounds retain the pharmaceutical effect in the distillate. The oily as well as the aqueous fraction of the steam distillate may be used as the product for manufacturing the pharmaceutical composition. In a preferred aspect, the oily steam distillate is used as the product. Thus, in the present invention, the term mastic oil describes the oily substance derived by steam distillation of the resin of the plant *Pistachia lentiscus* var. *chia.* This product includes all the essential oils of the plant.

In a certain aspect of the invention, the pharmaceutical composition is used for preventing inflammation. Thus, the pharmaceutical composition may be administered prior to exposure to a possible inflammatory agent.

In an aspect of the invention, the pharmaceutical composition comprises the product in a dilution of 10⁻⁵, preferably 10⁻⁶, or less in order to avoid any cytostatic or cytotoxic effect on the person treated with the pharmaceutical composition. Surprisingly, it has been shown that even at dilutions as low as around 10⁻⁷ the inflammatory effect is pronounced.

The pharmaceutical composition according to the present invention may be used as anti-inflammatory agent in local or generalised inflammatory conditions. In a certain aspect of the invention the pharmaceutical composition is formulated for topic administration, including epidermal, diadermal, or mucosal administration.

The product derived from the plant *Pistachia lentiscus* var. *chia* is suitable formulated as an ointment, a gel, or a cream when epidermal or diadermal administration is anticipated. However, other methods can be imagined like rags soaked with a solution of the product. The pharmaceutical composition, when adapted for mucosal administration through the mucosa in the mouth, may be formulated e.g. as a chewing gum, drops or a dragée.

For generalized inflammatory conditions it may be suitable to administer the product derived from *Pistachia lentiscus* var. *chia* by intravenous administration. Intravenous administering includes infusion and injection of a solution containing the product to be used according to the invention.

The invention also relates to a use of a product derived from the plant *Pistachia lentiscus* var. *chia* for the manufacture of a pharmaceutical composition for inhibiting proliferation of cells. The use comprises administering to a person in need thereof an amount of a product derived from the plant *Pistachia lentiscus* var. *chia* effective to inhibit proliferation of cells. In a certain embodiment, the cells to be inhibited in their proliferation is cancer cells, such as breast cancer cells, prostate cells, and cells from hepatocellular carcinoma. When the effect of inhibiting the proliferation of cells is desired, the product derived from the plant *Pistachia lentiscus* var. *chia* is used in a dilution of 10⁻⁵ or more, preferably 10⁻⁴ or more and most preferred 10⁻³ or more.

The invention further relates to a pharmaceutical composition comprising a product derived from the plant *Pistachia lentiscus* var. *chia* in a dilution of 10⁻⁵ or less, preferably 10⁻⁶ or less, and most preferred 10⁻⁷ or less. The solvent used for the dilution may be any solvent suitable for the purpose. Preferably however, the solvent is aqueous. Minor amounts of organic solvents may be present.

A suitable method of preparing the diluted mastic oil would be initially to dissolve the pure mastic oil in an organic solvent tolerable by the human body and miscible with water. Examples of such solvents include ethanol, acetone, and ethylene glycol. Subsequently, the initially dissolved product is further diluted using an aqueous media to obtain the final dilution.

### Brief disclosure of the drawings

Fig. 1 shows a correlation between the mastic oil dilution and the cytotoxic effect on various epithelial cell lines.
Fig. 2 depicts the effect of mastic oil on various stages of a cell cycle.

### Detailed description of the invention

In an experiment supporting the invention, the possible cytotoxic/cytostatic effect of mastic oil was assayed on a number of human tumour-derived cell lines. This was done in view of its possible use as an anti-inflamatory agent in humans. Lack of cytotoxicity is advantageous when the pharmaceutical composition is intended for the treatment of other indications. It was found, that only low dilutions (high concentrations) of mastic oil are toxic. This result limits the use of pure mastic oil in men. In subsequent experiments, in which dilutions 1/10,000,000 (10⁻⁷) were used, no cytotoxic effects were found. This result was also verified by assaying the effect of mastic oil on the cell cycle, which reflects the cytostatic and pro-apoptotic effects of a given agent.

In another experiment, isolated normal human leucocytes were used, onto which an experimental inflammation was initiated by lipopolysaccharide (LPS) and phytohemagglutinin (PHA). The production and release of pro-inflamatory cytokines were assayed at different time-frames from 1-24 hours. The results show that a pre-incubation of cells with mastic oil induces a substantial decrease of cytokine secretion, both at short (<12 hours) and long (>12 hours) incubation times.

In another experiment, immunoglobulin production (IgG, IgM, IgA, IgE) from isolated human B-lymphocytes was assayed, after pre-incubation of cells with mastic oil. It was found that the agent does not modify (at least at the dilutions used) the production of immunoglobulins, indicative of its anti-inflamatory action.

In a final experiment, we have assayed the activation of different cell populations, and its possible modification by mastic oil. We show that pre-incubation of cells with mastic oil decreased the activation of different cell populations (especially T- and B-lymphocytes), indicative of a protective effect of the agent against inflammatory processes. In contrast, after long incubation times, an activation of NK cells is observed.

Additional explanations of the invention are given in the following examples, which are not to be regarded as limiting the protection provided by the claims.

### Examples

### Example 1

### Action of mastic oil on cell proliferation of human epithelial cells

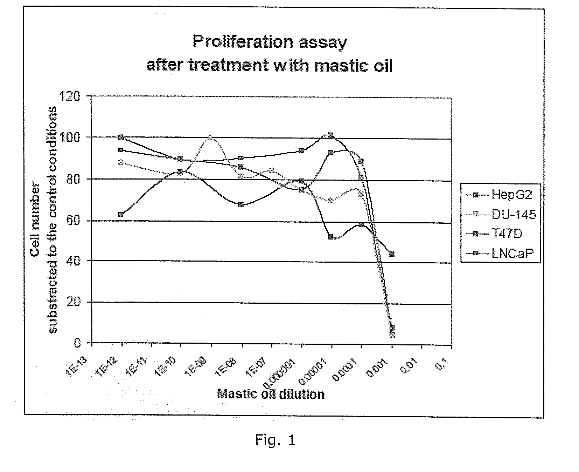

Pure mastic oil was diluted in absolute ethanol (1/100 %v/v, dilution 10⁻²) and subsequently in culture medium. The human epithelial cell lines T47D (from breast cancer, hormone sensitive), LNCaP and DU145 (from prostate cancer, hormone sensitive and resistant respectively) and HepG2 (from hepatocellular carcinoma) were tested. All these cell lines represent early stages of the corresponding diseases, retaining the majority of characteristics of their corresponding normal counterparts. The effect of mastic oil was assayed after two cell cycles. Our results are presented in Figure 1. As shown, the agent differentially affects the proliferative capacity of each cell line. In breast and prostate cell lines, the inhibitory effect of mastic oil attains 20-60%. However, this inhibitory effect does not exhibit the traditional sigmoidal curve in log-normal coordinates. Local maximal actions were found at dilutions 10⁻⁸ and 10⁻⁵. This effect may be explained by the presence in the complex extract of a number of substances acting cooperatively or competitively. In this respect, when a given compound attains its critical concentration express the full spectrum of its action. Similar results have also been found in other complex extracts from biological sources. These results indicate that mastic oil might be cytotoxic at dilutions lower than 1/10,000 (10⁻⁵), acting differentially on each specific cellular target, and therefore influencing in a discrete way each organ. It derives that, in a therapeutically use in humans, concentrations of mastic oil should not exceed this dilution factor, unless the cytoxic effect is desired.

### Example 2

### Effect of mastic oil on cell cycle

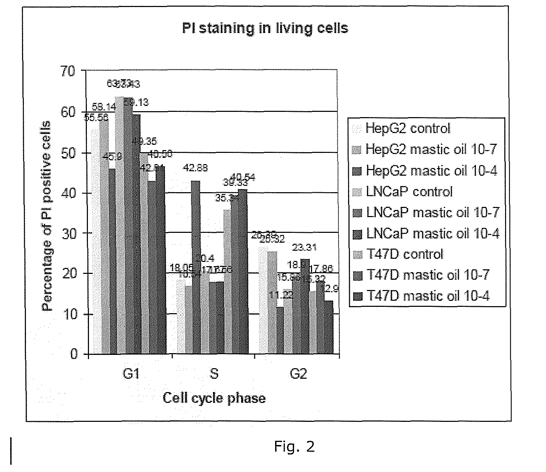

Another way to access a possible cytotoxicity of mastic oil or its constituents is the induction of apoptotic or necrotic cell death. Measurement of cell cycle phases may provide valid results on these phenomena. We have therefore incubated cells for 2 days, with two different dilutions of mastic oil: one (1/10,000,000; 10⁻⁷) had no effect on cell proliferation, while at the other (1/10,000; 10⁻⁴) cell proliferation is affected, as shown in Figure 2. Subsequently, cells were fixed and, after staining of DNA with propidium iodide, they were analyzed by flow cytometry.

In correspondence with the results of cell proliferation, hepatocytes seem not to be influenced by a dilution of 10⁻⁷ mastic oil. In contrast, lower dilutions (10⁻⁴) induce a dramatic increase in S phase, and a concomitant decrease of G1 and G2 phases. Accumulation of cells in S phase induces a loss of their proliferative capacity and induction of cell death.

### Example 3

### Anti-inflamatory action of mastich oil

Total leucocytes from a healthy blood donor were collected after venipuncture (150 ml) and lymphoprep^{®} density gradient centrifugation. Leucocyte layer was washed twice with normal saline and analyzed by flow cytometry, after identifying different cell populations (B- and T-lymphocytes, NK cells, monocytes, granulocytes) with fluorescent antibodies. This analysis showed that expected concentrations of all cell populations are isolated. Only monocytes were reduced, as expected, due to their capacity to adhere to a number of substrates. Thereafter, leucocytes were cultured, following standard experimental protocols.

Experimental inflammation was induced by lipopolysaccharide (LPS) and phytohemagglutinin (PHA) treatment The experimental protocol consisted to the measurement of a number of cytokines, after induction of inflammation (control conditions). Two different dilutions of mastic oil were tested (10⁻⁴ and 10⁻⁷), under two different experimental protocols: Pre-incubation with mastic oil before the induction of inflammation, or introduction its introduction during with the pro-inflamatory agents. The experimental protocol is presented in the following table.

**Table 2**

| Hours of sampling | -1 | 0 | 1 | 2 | 6 | 12 | 24 |
|---|---|---|---|---|---|---|---|
| Basal conditions | + | + | + | + | + | + | + |
| Inflammation | | + | + | + | + | + | + |
| Mastich oil 10⁻⁴ | + | + | + | + | + | + | + |
| Mastich oil 10⁻⁷ | + | + | + | + | + | + | + |
| Inflammation + Mastich oil 10⁻⁴ | | + | + | + | + | + | + |
| Inflammation + Mastich oil 10⁻⁷ | | + | + | + | + | + | + |
| Mastich oil 10⁻⁴ + Inflammation | | + | + | + | + | + | + |
| Mastich oil 10⁻⁷ + Inflammation | | + | + | + | + | + | + |

The production of a number of pro-inflamatory cytokines was assayed at different time points, from 1-24 hours. It has to be noted that incubation of cells for 24 hours with purified extracts in vitro is comparable to a chronic (at least one week) administration of the agents in vivo.

As presented in the following Tables, our results show that pre-incubation (both for short and long time periods) with mastic oil before the induction of inflammation, decreases significantly the secretion of pro-inflamatory cytokines, indicating a protective anti-inflamatory action of the agent. In contrast, application of mastic oil after the induction of inflammation has a reduced efficiency. This effect may be due either to a decreased efficiency of mastic oil after the induction of inflammation per se, or to the maximal stimulation of leucocytes, decreasing their response to other agents.

**Preincubation Mastich**

| | Macrophages | CD4 | | | CD8 | B cells | NK | Granulocytes | Stroma cells |
|---|---|---|---|---|---|---|---|---|---|
| | | Th0 | TH1 | TH2 | | | | | |
| IL1 | Decreased activation | | | | | | | | |
| IL2 | | Decreased secretion | Decreased differentiation | Decreased differentiation | | | | | |
| IL4 | | | | Decreased secretion | | | | | |
| IL5 | | | | Decreased secretion | | | | | |
| IL6 | Decreased secretion | | | Decreased secretion | | | | | |
| IL7 | | | | | | | | | No effect |
| IL8 | Decreased secretion <2h | | | | | | | | |
| IL10 | | | | Decreased secretion | | | | | |
| IL12 | Decreased secretion <16h | | | | | | | | |
| IL13 | | | | Decreased secretion | | | | | |
| INFγ | | | Decreased secretion | | | | | | |
| GM-CSF | | | Decreased secretion | | | | | | |
| TNFα | Decreased secretion <4h | | | | | | | | |

**Preincubation LPS**

| | Macrophages | CD4 | | | CD8 | B cells | NK | Granulocytes | Stroma cells |
|---|---|---|---|---|---|---|---|---|---|
| | | Th0 | TH1 | TH2 | | | | | |
| IL1 | Increased secretion 4h | | | | | | | | |
| IL2 | | Decreased secretion >4h | | | | | | | |
| IL4 | | | | Decreased secretion | | | | | |
| IL5 | | | | Decreased secretion | | | | | |
| IL6 | Increased secretion | | | Increased secretion | | | | | |
| IL7 | | | | | | | | | Increased secretion |
| IL8 | No effect | | | | | | | | |
| IL10 | | | | Decreased secretion | | | | | |
| IL12 | Decreased secretion <16h | | | | | | | | |
| IL13 | | | | Decreased secretion | | | | | |
| INFγ | | | Decreased secretion | | | | | | |
| GM-CSF | | | Decreased secretion | | | | | | |
| TNFα | No effect | | | | | | | | |

**Preincubation Mastich**

| | Macrophages | CD4 | | | CD8 | B cells | NK | Granulocytes | Stroma cells |
|---|---|---|---|---|---|---|---|---|---|
| | | Th0 | TH1 | TH2 | | | | | |
| IL1 | Decreased activation | | | | | | | | |
| IL2 | | Decreased secretion | Decreased differentiation | Decreased differentiation | | | | | |
| IL4 | | | | No effect | | | | | |
| IL5 | | | | No effect | | | | | |
| IL6 | ? | | | ? | | | | | |
| IL7 | | | | | | | | | No effect |
| IL8 | Decreased secretion | | | | | | | | |
| IL10 | | | | Decreased secretion | | | | | |
| IL12 | No effect | | | | | | | | |
| IL13 | | | | No effect | | | | | |
| INFγ | | | No effect | | | | | | |
| GM-CSF | | | No effect | | | | | | |
| TNFα | Increased secretion | | | | | | | | |

**Preincubation LPS**

| | Macrophages | CD4 | | | CD8 | B cells | NK | Granulocytes | Stroma cells |
|---|---|---|---|---|---|---|---|---|---|
| | | Th0 | TH1 | TH2 | | | | | |
| IL1 | No effect | | | | | | | | |
| IL2 | | No effect | | | | | | | |
| IL4 | | | | Increased secretion | | | | | |
| IL5 | | | | Increased secretion | | | | | |
| IL6 | ? | | | ? | | | | | |
| IL7 | | | | | | | | | No effect |
| IL8 | No effect | | | | | | | | |
| IL10 | | | | Decreased secretion | | | | | |
| IL12 | No effect | | | | | | | | |
| IL13 | | | | Increased secretion | | | | | |
| INFγ | | | No effect | | | | | | |
| GM-CSF | | | No effect | | | | | | |
| TNFα | No effect | | | | | | | | |

**Preincubation Mastich**

| | Macrophages | CD4 | | | CD8 | B cells | NK | Granulocytes | Stroma cells |
|---|---|---|---|---|---|---|---|---|---|
| | | Th0 | TH1 | TH2 | | | | | |
| IL1 | Decreased activation | | | | | | | | |
| IL2 | | Decreased secretion | Decreased differentiation | Decreased differentiation | | | | | |
| IL4 | | | | Decreased secretion | | | | | |
| IL5 | | | | Decreased secretion | | | | | |
| IL6 | Decreased secretion | | | Decreased secretion | | | | | |
| IL7 | | | | | | | | | No effect |
| IL8 | Decreased secretion | | | | | | | | |
| IL10 | | | | Decreased secretion | | | | | |
| IL12 | Decreased secretion <16h | | | | | | | | |
| IL13 | | | | Decreased secretion | | | | | |
| INFγ | | | No effect | | | | | | |
| GM-CSF | | | No effect | | | | | | |
| TNFα | No effect | | | | | | | | |

**Preincubation LPS**

| | Macrophages | CD4 | | | CD8 | B cells | NK | Granulocytes | Stroma cells |
|---|---|---|---|---|---|---|---|---|---|
| | | Th0 | TH1 | TH2 | | | | | |
| IL1 | No effect | | | | | | | | |
| IL2 | | No effect | | | | | | | |
| IL4 | | | | Decreased secretion | | | | | |
| IL5 | | | | Decreased secretion | | | | | |
| IL6 | Increased secretion | | | Decreased secretion | | | | | |
| IL7 | | | | | | | | | Increased secretion |
| IL8 | Decreased secretion | | | | | | | | |
| IL10 | | | | Decreased secretion | | | | | |
| IL12 | Decreased secretion <16h | | | | | | | | |
| IL13 | | | | Decreased secretion | | | | | |
| INFγ | | | Decreased secretion | | | | | | |
| GM-CSF | | | Decreased secretion | | | | | | |
| TNFα | No effect | | | | | | | | |

### Example 4

### Effect of mastic oil on the production of immunoglobulins by isolated lymphocytes

Leucocyte isolation and the control of their purity was made as per Example 3. Pre-incubation with mastic oil for 12 hours at dilutions 10⁻⁴ and 10⁻⁷ followed, and immunoglobulins were measured with a specific nefelometric method. Our results show that mastich oil does not induce the secretion of either IgG, IgM, IgE or IgA. In contrast, experimental inflammation increases dramatically all four immunoglobulins.

### Example 5

### Activation of leucocyte sub-population by mastic oil

By the use of targeting antibodies against specific surface molecules of naive or activated cell populations, we assayed the activation of different cells under basic conditions or after induction of experimental inflammation, and accessed the effect of mastic oil. As for cytokine production, pre-incubation of cells with mastic oil decreased the activation of a number of cell populations (especially T- and B-lymphocytes), a result suggestive of a selective anti-inflamatory action of the agent. In contrast, pre-incubation of cells with mastic oil alone, induces after long incubation times, the activation of NK cells.

## Claims

1. Use of a product derived from the plant *Pistachia lentiscus* var. *chia* for the manufacture of a pharmaceutical composition for treating or preventing inflamation.

2. Use according to claim 1, wherein the said product is the resin of the plant *Pistachia lentiscus* var. *chia,*

3. Use according to claim 1 or 2, wherein the said product is the oily steam distillate of the resin.

4. Use according to any of the claims 1-3, wherein the pharmaceutical composition comprises the product in a dilution of 10⁻⁵ or less.

5. Use according to claim 4, wherein the pharmaceutical composition comprises the product in a dilution of 10⁻⁶ or less.

6. Use according to any of the claims 1-5, wherein the inflamation treated or prevented by the pharmaceutical composition is a local or generalized inflammatory condition.

7. Use according to any of the claims 1-6, wherein the pharmaceutical composition is formulated for epidermal, diadermal, or mucosal administration.

8. Use according to claim 7, wherein the pharmaceutical composition for epidermal or diadermal administration comprises an ointment, a gel, or a cream.

9. Use according to claim 7, wherein the pharmaceutical composition for mucosal administration comprises chewing gum and dragee.

10. Use according to any of the claims 1 to 6, wherein the pharmaceutical composition is for intravenous, oral, rectal, parenteral, subcutaneous, intradermal, intramuscular or intravenous administering.

11. Use of a product derived from the plant *Pistachia lentiscus* var. *chia* for the manufacture of a pharmaceutical composition for inhibiting proliferation of cells.

12. Use according to claim 11, wherein the cells to be inhibited in proliferation include cancer cells.

13. A pharmaceutical composition comprising a product derived from the plant *Pistachia lentiscus* var. *chia* in a dilution of 10⁻⁵ or less.

14. The pharmaceutical composition according to claim 13, wherein the dilution is 10⁻⁶ or less.

15. The pharmaceutical composition according to claim 13, wherein the dilution is 10⁻⁷ or less.

16. The pharmaceutical composition according to any of the claims 13 to 15, wherein the said product is the resin of the plant *Pistachia lentiscus* var. *chia.*

17. The pharmaceutical composition according to any claims 13 to 16, wherein the said product is the oily steam distillate of the resin or the resulting aqueous phase.

18. The pharmaceutical composition according to any of the claims 13 to 17, wherein the solvent used for the dilution is any chemically acceptably organic or inorganic solvent.

19. The pharmaceutical composition according to claim 18, wherein the solvent is aqueous.
